# EUROPEAN PATENT APPLICATION

(11) **EP 2 742 954 A1**
(43) Date of publication of application: **18.06.2014**
(21) Application number: 12197427.3
(22) Date of filing: 17.12.2012
(51) Int. Cl.: A61K 47/10, A61K 9/08, A61K 9/10, A61K 31/00, A61K 36/00

(54) **"Silymarin aqueous formulation"**

(71) Applicant: INDENA S.p.A., 20139 Milano (IT)
(72) Inventor: Baghi, Daniele, 20139 MILANO Italy (IT); Milano, Ettore, 20139 MILANO Italy (IT); Satoshi, Watanabe, Hadano, Kanagawa 259-1304 (JP)
(74) Representative: Barchielli, Giovanna

(57) **Abstract**

The present invention relates to formulations aqueous di silymarin containing surfactant or a mixture of surfactants having a value of HLB higher than 8 and a polyol or a mixture of polyols. Such formulations can be subjected to heating, in particular a pasteurization process, without formation of aggregates.

## Description

The present invention relates to an aqueous formulation of silymarin, to a process for its preparation and to the use of said formulation for the prevention and/or the treatment of liver damage.

Silymarin is the standardized extract of the fruit of the milk thistle plant (Silybum marianium) and it contains substantially three flavonoids of the flavonol subclass: silybin, which is predominant, the silydianin and the silychristin.

Silymarin is known to have hepatoprotecting and depurating properties and it is used as adjuvant in the treatment of the liver damage (Malandrino et al. Drugs Future 1990; 15:226-227, Parris Kidd et al. Alternative Medicine Review Vol. 10 (3) 2005, 193-203).

The silymarin is sparingly water-soluble and has a poor bioavailability. The aqueous dispersions of silymarin tend to form aggregates particularly under heating conditions. This problem becomes particularly relevant during the pasteurization process of beverages containing silymarin when the aqueous dispersion is subjected to temperatures of about 80°C.

It has now been found that the aqueous formulations of silymarin, containing hydrophilic surfactants having middle to high HLB (Hydrophilic Lipophilic Balance) and polyols, do not form aggregates when they are subjected to heating.

An object of the present invention is an aqueous formulation of silymarin containing:
a) a surfactant or a mixture of surfactants having a value of HLB higher than 8 and
b) a polyol or a mixture of polyols.

Preferably the surfactant or the mixture of surfactants has a value of HLB higher than 12.

The surfactants are preferably selected from the group consisting of glycerol esters of fatty acids, sorbitan esters, polysorbates, sucrose esters, lecithins or mixtures thereof.

Glycerol esters of fatty acids are preferably decaglycerol esters of a fatty acid, particularly preferred is decaglycerol monooleate.

Sorbitan esters may be for example sorbitan monolaurates.

Sucrose esters are sucrose esters of a fatty acid, preferred is sucrose monopalmitate.

Polysorbates are preferably polyoxyethylene (20) sorbitan esters of a fatty acid. Particularly preferred is polyoxyethylene (20) sorbitan monostealates.

Preferred lecithins are soybean lecithin or a lysolecithin, in particular defatted lysolecithin.

Preferred polyols utilizable in the formulation of the invention are propylene glycol, glycerol, butanediol, pentandiol, hexandiol and a sugar alcohol or a mixture thereof.

Particularly preferred is glycerol.

The aqueous formulation of the invention may optionally contain ethanol.

The concentration of silymarin in the formulations of the invention preferably is from 0.01 to 10% by weight.

The surfactant or the mixture of surfactants is preferably present in a concentration from 0.01 to 40% by weight.

The polyol or the mixture of polyols is preferably present in a concentration from 1 to 99% by weight.

When present ethanol has a concentration from 0.2 to 20% by weight.

A preferred aqueous formulation according the invention contains:

| | |
|---|---|
| i) silymarin | 0.01-10% by weight |
| ii) soy bean lecithin | 0.01-10% by weight |
| iii) ethanol | 0.2-20% by weight |
| iv) glycerol | 1-99% by weight |
| v) decaglycerol monooleate | 0.02-40% by weight |
| vi) water | 0-98% by weight |

The aqueous formulations according to the invention are prepared according to a process comprising the steps:
   a) dissolving silymarin in a polyol or a polyol mixture and optionally ethanol;
   b) dissolving soy bean lecithin and the hydrophilic surfactant in a mixture of water and the residual polyol;
   c) dissolving the silymarin solution obtained in step a) in the emulsifier solution obtained in step b);
   d) dissolving the resulting solution in water;
   e) pasteurizing the obtained aqueous solution;

### Examples:

### Example 1

Aqueous formulation containing:

| | |
|---|---|
| Silymarin ET | 5.0% by weight |
| Glycerol | 45.0% by weight |
| Decaglycerol monooleate | 25.0% by weight |
| Ethanol (99.5 vol%) | 10.0% by weight |
| Soy bean lecithin | 5.0% by weight |
| Water | 10.0% by weight |
| pH | 3.0 |

Process:
Silymarin was dissolved in a mixture ethanol/glycerol at 60°C to obtain the "silymarin solution". The surfactants, decaglycerol monooleate and the soy bean lecithin were dissolved in a mixture of water and the residual glycerol at 80°C to obtain the "emulsifier solution".

The "silymarin solution" was dissolved in the "emulsifier solution" at a temperature under 50°C.

The obtained composition was dispersed in water with the formation of an emulsion wherein the micelles have a mean diameter of 100-200 nm. The aqueous emulsion was pasteurized at 80°C for 30 minutes. No change was observed in the appearance and in the mean diameter of the micelles.

### Example 2 (Comparative example)

Aqueous solutions of glycerol (45% by weight), ethanol (10% by weight), decaglycerol monooleate (10% by weight) and soy bean lecithin (5% by weight) were prepared. Silymarin (5% by weight) was dispersed in each of the above solutions. The obtained dispersions of silymarin were heated in order to detect the formation of aggregates.

| **Solution** | **Appearance change** |
|---|---|
| Glycerol | aggregation at 40°C |
| Ethanol | aggregation at 40°C |
| Decaglycerol monooleate | aggregation at 40°C |
| Soy bean lecithin | slight aggregation a 80°C |

### Example 3

Aqueous formulation containing:

| | |
|---|---|
| Silymarin | 5% by weight |
| Ethanol | 10% by weight |
| Glycerol | 45% by weight |
| Decaglycerol monooleate | 30% by weight |
| Water | 10% by weight |

The aqueous solution was obtained by the process described in example 1.

### Example 4

Aqueous formulation containing:

| | |
|---|---|
| Silymarin | 5% by weight |
| Ethanol | 10% by weight |
| Glycerol | 45% by weight |
| Defatted lysolecithin | 30% by weight |
| Water | 10% by weight |

The aqueous solution was obtained by the process described in example 1.

### Example 5

Aqueous formulation containing:

| | |
|---|---|
| Silymarin | 5% by weight |
| Glycerol | 55% by weight |
| Defatted lysolecithin | 30% by weight |
| Water | 10% by weight |

The aqueous solution was obtained by the process described below.

Glycerol and defatted lecithin were mixed and heated. Silymarin was added to the mixture and dissolved by heating. Water was added to obtain the aqueous solution.

The obtained composition was dispersed in water with the formation of an emulsion wherein the micelles have a mean diameter of 100-200 nm. The aqueous emulsion was pasteurized at 80°C for 30 minutes. No change was observed in the appearance and in the mean diameter of the micelles.

### Example 6

Aqueous formulation containing:

| | |
|---|---|
| Silymarin | 5% by weight |
| Ethanol | 2% by weight |
| Glycerol | 53% by weight |
| Defatted lysolecithin | 30% by weight |
| Water | 10% by weight |

The aqueous solution was obtained by the process described in example 1.

## Claims

1. An aqueous formulation of silymarin containing:
a) a surfactant or a mixture of surfactants having a value of HLB higher than 8 and
b) a polyol or a mixture of polyols.

2. The aqueous formulation of silymarin according to claim 1 wherein the surfactant or the mixture of surfactants has a HLB higher than 12.

3. The aqueous formulation according to claim 1 or 2 wherein the surfactant is selected from the group consisting of glycerol esters of fatty acids, sorbitan esters, polysorbates, sucrose esters, lecithins or mixtures thereof.

4. The aqueous formulation according to claim 3 wherein the glycerol esters of fatty acid is a decaglycerol ester of a fatty acid.

5. The aqueous formulation according to claim 4 wherein the decaglycerol ester of a fatty acid is decaglycerol monooleate.

6. The aqueous formulation according to claim 5 wherein the lecithin is soybean lecithin or a lysolecithin.

7. The aqueous formulation according to claim 6 wherein the lysolecithin is a defatted lysolecithin.

8. The aqueous formulation according to claims 1-7 wherein the polyol is selected from the group consisting of propylene glycol, glycerol, butanediol, pentandiol, hexandiol and a sugar alcohol or a mixture thereof.

9. The aqueous formulation according to claim 8 wherein the polyol is glycerol.

10. The aqueous formulation according to each of the claims from 1 to 9 further containing ethanol.

11. The aqueous formulation according to claims 1-10 wherein silymarin has a concentration from 0.01 to 10% by weight.

12. The aqueous formulation according to claims 1-11 wherein the surfactant or the mixture of surfactants has a concentration from 0.01 to 40% by weight.

13. The aqueous formulation according to claims 1-12 wherein the polyol or the mixture of polyols has a concentration from 1 to 99% by weight

14. The aqueous formulation according to claim 10 wherein ethanol has a concentration from 0.2 to 20% by weight.

15. An aqueous formulation containing:
| | |
|---|---|
| i) silymarin | 0.01-10% by weight |
| ii) soy bean lecithin | 0.01-10% by weight |
| iii) ethanol | 0.2-20% by weight |
| iv) glycerol | 1-99% by weight |
| v) decaglycerol monooleate | 0.02-40% by weight |
| vi) water | 0-98% by weight |

16. The aqueous formulation according to claim 1 for the prevention and/or the treatment of liver damage.
